# EUROPEAN PATENT APPLICATION

(11) **EP 4 209 224 A1**
(43) Date of publication of application: **12.07.2023**
(21) Application number: 21862130.8
(22) Date of filing: 27.08.2021
(51) Int. Cl.: A61K 36/185, A61P 3/10, A23L 33/105, A23K 10/30

(54) **COMPOSITION FOR IMPROVING INSULIN SENSITIVITY, CONTAINING GENTIANA TRIFLORAL PALLAS EXTRACT**

(30) Priority: 28.08.2020 KR 20200109373
(71) Applicant: Mthera Pharma Co., Ltd., Seoul 04779 (KR); Apharma, Goyang-si Gyeonggi-do 10326 (KR)
(72) Inventor: SOHN, Mi Won, Yongin-si Gyeonggi-do 16822 (KR); KIM, Sinyeon, Seoul 07063 (KR); CHOI, Jin Gyu, Seoul 03505 (KR); KIM, Se Woong, Seoul 07522 (KR); PARK, Sang Cheol, Seoul 03115 (KR); CHOI, Ji Seon, Seoul 08634 (KR); LEE, Jin Su, Seoul 02787 (KR)
(74) Representative: Duncombe, Jessica
(86) International application number: PCT/KR2021/011530
(87) International publication number: WO 2022/045834

(57) **Abstract**

The present invention relates to a composition for improving insulin sensitivity, comprising a *Gentiana triflora Pallas* extract. The composition according to the present invention improves insulin sensitivity by reducing the expression of ITIH1, and thus can be effectively used as a therapeutic agent for diabetes, diabetic complications, impaired glucose tolerance, insulin resistance syndrome and the like.

## Description

### Technical Field

The present invention relates to a composition for improving insulin sensitivity, comprising a *Gentiana triflora Pallas* extract. More particularly, the present invention relates to a composition characterized by improving insulin sensitivity by reducing the expression of ITIH1 (Inter-alpha Trypsin Inhibitor Heavy chain 1).

### Background Art

In normal cases, the body maintains homeostasis by regulating blood glucose concentration. However, in metabolic diseases and diseases accompanied by insulin resistance, blood sugar is excessively increased, and when such high blood sugar persists, tissue damage and functional disorders are caused.

Insulin resistance is a decrease in sensitivity to insulin compared to the normal standards, and refers to a case in which an increase in insulin in muscle and adipose tissue is not detected or the action of insulin does not occur effectively even if it is detected. In this case, the balance of cell metabolism and metabolism cannot be effectively maintained because blood sugar cannot be lowered.

In order to improve insulin sensitivity, a method of increasing the secretion of insulin itself in the beta (β) cells of the pancreas or amplifying the signal transmitted by the insulin receptor binding to insulin in the metabolic pathway is mainly considered, but research on how to control hyperglycemia by regulating the activity of the protein itself involved in hyperglycemia based on the biological mechanism that causes hyperglycemia is insufficient.

ITIH1 (Inter-alpha Trypsin Inhibitor Heavy chain 1) is a protein produced in hepatocytes and secreted into the blood, and its intracellular concentration and secretion amount are increased by glucose stimulation (Science Translational Medicine, Vol. 11, Issue 513, eaan4735, 2019). Specifically, the increase in ITIH1 is achieved by a decrease in G13, a type of G protein, and an increase in the expression level of OGT (O-GlcNAc transferase) enzyme in hyperglycemia. In a hyperglycemic environment, OGT induces O-GlcNAcylation modification on the serine residue of ITIH1, which increases the stability of ITIH1. As a result, when the concentration of ITIH1 is increased, ITIH1 binds to hyaluronic acid and reduces insulin sensitivity in muscle and adipocytes.

On the other hand, *Gentiana triflora Pallas* is a perennial plant of the *Gentiana scabra* family, and its flowers bloom in sky blue in July and August, the rhizome is thick, the main stem stands straight, and there is no hair. It is distributed in Korea, Manchuria, Japan, and eastern Siberia. It is similar to *Gentiana scabra,* but it is different from *Gentiana scabra* in that there are no projections on the stem and the main veins on the lower surface of leaves, the flowers do not bloom in full bloom, and the forked pieces of the corolla are not bent. The roots that are dug up and dried in the fall are used for indigestion, cholecystitis, jaundice, headache, encephalitis, cystitis, urethritis, and the like, but nothing is known about an effect of *Gentiana triflora Pallas* on improving insulin sensitivity.

In this regard, the inventors of the present invention found that a *Gentiana triflora Pallas* extract can improve insulin sensitivity by inhibiting the expression of ITIH1. Based on the above, the present inventors completed the present invention.

### Prior Art Document

### Non-Patent Document

(Non-Patent Document 1) Science Translational Medicine, Vol. 11, Issue 513, eaan4735 (2019.10.09)

### Detailed Description of Invention

### Technical Problem

An object of the present invention is to provide a composition capable of improving insulin sensitivity by reducing the expression of ITIH1, comprising a *Gentiana triflora Pallas* extract.

### Solution to Problem

In order to achieve the above object, the present invention provides a composition for improving insulin sensitivity, comprising a *Gentiana triflora Pallas* extract.

The composition may be a pharmaceutical composition, a food composition, or an animal feed composition.

In one embodiment, the composition has an effect of increasing insulin sensitivity and reducing insulin resistance.

In one embodiment, the composition may be for the prevention, treatment or improvement of at least one disease selected from the group consisting of diabetes, diabetic complications, impaired glucose tolerance, and insulin resistance syndrome.

In one embodiment, the diabetic complications may be at least one disease selected from the group consisting of diabetic retinopathy, diabetic cataract, diabetic nephropathy, and diabetic neuropathy.

In one embodiment, the insulin resistance syndrome may be at least one disease selected from the group consisting of obesity, hypertension, arteriosclerosis, hyperlipidemia, hyperinsulinemia, non-alcoholic fatty liver, and type 2 diabetes caused by insulin resistance.

In one embodiment, the *Gentiana triflora Pallas* extract may be a *Gentiana triflora Pallas* whole plant extract, a root extract, a leaf extract, a flower extract or a stem extract, and preferably, it may be a *Gentiana triflora Pallas* whole plant extract or a *Gentiana triflora Pallas* root extract.

In one embodiment, the *Gentiana triflora Pallas* extract may reduce the expression of *itih1* (Inter-alpha Trypsin Inhibitor Heavy chain 1) mRNA or ITIH1 protein.

In one embodiment, the *Gentiana triflora Pallas* extract may reduce the expression *of ogt* (O-GlcNAc Transferase) mRNA.

In one embodiment, the extract may be extracted with a solvent selected from the group consisting of water, C₁ to C₆ alcohol, and mixed solvents thereof, and may be a methanol extract.

In one embodiment, the extract may be extracted with an aqueous solution of methanol 10 times the weight of *Gentiana triflora Pallas,* but is not limited thereto.

In one embodiment, the extract may be ultrasonically extracted at 40 to 50 °C, but is not limited thereto.

### Effects of Invention

The composition comprising a *Gentiana triflora Pallas* extract according to the present invention has an effect of improving insulin sensitivity by reducing the expression of ITIH1. Therefore, the composition of the present invention can be effectively used as a therapeutic agent for diabetes, diabetic complications, impaired glucose tolerance, insulin resistance syndrome and the like.

### Brief Description of Drawings

Figure 1 shows the cell cytotoxicity of a *Gentiana triflora Pallas* extract.
Figure 2 shows the increase in *itih1* mRNA expression under high concentration glucose conditions.
Figure 3 shows an effect of inhibiting *itih1* mRNA expression when treated with a *Gentiana triflora Pallas* extract.
Figure 4 shows an effect of inhibiting ITIH1 protein expression when treated with a *Gentiana triflora Pallas* extract.
Figure 5 shows the increase in *ogt* mRNA expression when treated with MGO under high concentration glucose conditions.
Figure 6 shows an effect of inhibiting *ogt* mRNA expression when treated with a *Gentiana triflora Pallas* extract.

### Best Mode for Carrying out the Invention

Hereinafter, with reference to the accompanying drawings, embodiments and examples of the present invention will be described in detail so that those of ordinary skill in the art to which the present invention belongs can easily carry out. However, the present invention may be implemented in various forms and is not limited to the embodiments and examples described herein.

Throughout the present specification, when a certain part "includes" a certain component, it means that other components may be further included, rather than excluding other components, unless otherwise stated.

The present invention relates to a composition for improving insulin sensitivity, comprising a *Gentiana triflora Pallas* extract.

The *Gentiana triflora Pallas* extract of the present invention may be a *Gentiana triflora Pallas* whole plant extract, a root extract, a leaf extract, a flower extract or a stem extract. The whole plant refers to the entire plant with flowers, roots, leaves, stems, and the like.

The composition has the efficacy of inhibiting the expression of ITIH1 (Inter-alpha Trypsin Inhibitor Heavy chain 1). ITIH1 is a protein produced in hepatocytes and secreted into the blood, and its intracellular concentration and secretion amount are increased by glucose stimulation. It binds to hyaluronic acid and reduces insulin sensitivity in muscle and adipocytes.

In addition, the composition inhibits the expression of OGT (O-GlcNAc transferase). OGT increases the stability and concentration of ITIH1 by causing O-GlcNAcylation modifications to serine residues in the ITIH1 protein. Therefore, the composition of the present invention has an effect of improving insulin sensitivity by reducing the expression of ITIH1 and OGT.

The effect of improving insulin sensitivity refers to an increase in insulin sensitivity and a decrease in insulin resistance. Insulin sensitivity refers to the degree of response to insulin. A decrease in insulin sensitivity, i.e., insulin resistance, means that cells cannot effectively burn glucose due to a decrease in the response to insulin compared to the normal standards, resulting in a decrease in the function of insulin to lower blood sugar.

As a method for extracting the *Gentiana triflora Pallas* extract of the present invention, conventional methods in the art such as stirring extraction, filtration, hot water extraction, immersion extraction, reflux cooling extraction, and ultrasonic extraction can be used.

The pharmaceutical composition of the present invention can be parenterally administered or orally administered depending on the desired method, and the range of dosage varies depending on the patient's body weight, age, sex, health condition, diet, administration time, administration method, excretion rate, and severity of the disease and the like. In addition, the therapeutically effective amount of the composition may vary depending on the administration method, the target site, and the condition of the patient, and when used in the human body, the dosage should be determined in an appropriate amount in consideration of safety and efficiency.

The pharmaceutical composition of the present invention may be formulated and used in any form suitable for pharmaceutical preparations, including oral formulations such as powders, granules, tablets, soft or hard capsules, suspensions, emulsions, syrups, aerosols and the like, external formulations for skin such as ointments, creams and the like, suppositories, injections and sterile injection solutions and the like, respectively, according to conventional methods.

Excipients such as fillers, extenders, binders, wetting agents, disintegrants, surfactants, diluents, and the like commonly used for the formulation may be further included. For example, excipients that may be included in the pharmaceutical composition of the present invention may include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, methyl hydroxy benzoate, propyl hydroxy benzoate, talc, magnesium stearate and mineral oil and the like, but are not limited thereto. In addition to simple excipients, lubricants such as magnesium stearate and talc may be also used. Formulations for parenteral administration include sterilized aqueous solutions, non-aqueous solvents, suspensions, emulsions, freeze-dried formulations, and suppositories. Propylene glycol, polyethylene glycol, vegetable oils such as olive oil, and injectable esters such as ethyl oleate may be used as non-aqueous solvents and suspending agents. Witepsol, macrogol, tween 61, cacao butter, laurin butter, glycerogelatin, and the like may be used as a base for the suppository.

The food composition of the present invention may be a health functional food. The "health functional food" refers to a food manufactured and processed using raw materials or ingredients having useful functionality for the human body, and "functionality" refers to regulating nutrients for the structure and function of the human body or ingesting nutrients for the purpose of obtaining useful effects for health purposes such as physiological functions and the like.

The food composition can be manufactured and processed into health functional foods in the form of tea bags, leached tea, health drinks, and the like, and can be also processed into other breads, confectionaries, ice creams, noodles, and the like. In addition, the food composition may be a health supplement food or a food additive, and whether or not it is suitable as a "food additive" is determined by the standards and criteria for the relevant item in accordance with the General Rules of the Food Additives Code and General Test Methods and the like approved by the Ministry of Food and Drug Safety, unless otherwise specified.

Hereinafter, the present invention will be described in more detail through the examples, but the following examples are for illustrative purposes only and are not intended to limit the scope of the present invention.

### [Preparation Example]

### Preparation of Gentiana triflora Pallas extract

Ultrasonic extraction was performed at 45 °C by adding 10 folds of 99.9% methanol to 100 g of dried powder for each part of the whole plant and root of *Gentiana triflora Pallas* purchased from the Korea Plant Extract Bank. The process of operating the ultrasonic extractor for 15 minutes and stopping for 2 hours was defined as one time, and the process was performed a total of 30 times for 3 days to obtain an extraction solution. This extraction solution was filtered, and then it was concentrated under reduced pressure at 45 °C to obtain a powder, and the following experiments were conducted.

### [Example 1]

### Confirmation of cell cytotoxicity of Gentiana triflora Pallas extract

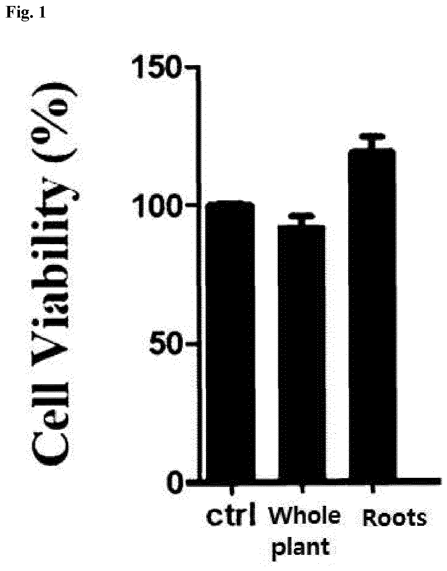

In order to confirm the cell cytotoxicity of the *Gentiana triflora Pallas* extract, MTT assay was performed as follows. the HepG2 cells were dispensed to 4×10⁴ cells/ml in a 96 well plate and then cultured for 24 hours to perform attachment and stabilization. After culturing for 24 hours, the *Gentiana triflora Pallas* whole plant, root extract was diluted in the culture solution to a final extraction concentration of 10 µg/ml, supplied to the cell line, and cultured for 24 hours. After 24 hours, the medium was removed, and 100 µl of the MTT solution (0.5 mg/ml in PBS) was dispensed and reacted for 30 minutes to reduce MTT in a carbon dioxide humidified incubator (CO₂ Incubator) at 37 °C, 5 % conditions. Formazan crystals generated in each well were dissolved in 200 µl of DMSO (dimethyl sulfoxide). The absorbance of each sample was measured at 570 nm using a multimode plate reader (Varioskan LUX multimode plate reader, Thermo Scientific, Vantaa, Finland), and cell viability was confirmed using the untreated group as a negative control, and the results are shown in Figure 1.

As shown in Figure 1, as a result of measuring the cell cytotoxicity in the HepG2 cell line, it was confirmed that all extracts of *Gentiana triflora Pallas* showed a cell viability of 90% or more compared to the control group, indicating no cell cytotoxicity.

### [Example 2]

### Confirmation of increased expression of itih1 mRNA under high concentration glucose conditions

It is known that ITIH1 binds to hyaluronic acid, deposits in hepatocytes, and reduces the insulin sensitivity of cells. The evaluation method for ITIH1 inhibitory efficacy was confirmed as follows.

Primary cells isolated from cell lines or animals were cultured in a cell culture medium containing low concentration (5 mM) and high concentration (25 mM) glucose for 24 hours, respectively. At this time, it was confirmed that the O-GlcNAc modification (CTD 110.6 clone) increased and the expression of ITIH1 increased in the medium containing high concentration of glucose.

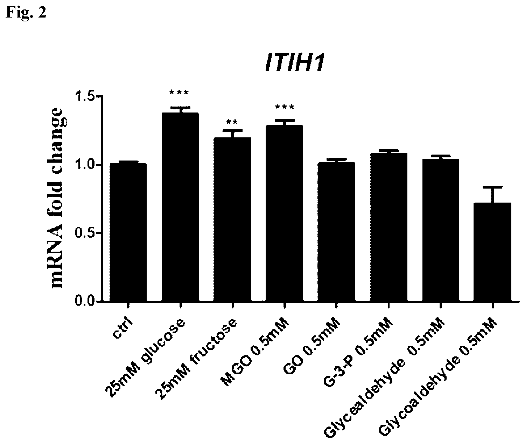

In addition, the HepG2 cells were cultured in a cell culture medium containing glucose, fructose, methylglyoxal (MGO), glyoxal (GO), glyceraldehyde 3-phosphate (G3P), glyceraldehyde, and glycolaldehyde for 24 hours, respectively, and the expression level of *itih1* mRNA was measured and shown in Figure 2.

As shown in Figure 2, the *itih1* mRNA expression was increased by about 1.5 times in cells cultured in a cell culture medium containing high concentration (25 mM) glucose compared to the normal group. Therefore, in the following, the expression of ITIH1 according to the treatment with the *Gentiana triflora Pallas* extract was confirmed at the mRNA and protein levels in the HepG2 cells grown in a high concentration glucose medium.

### [Example 3]

### Confirmation of effect of Gentiana triflora Pallas extract on inhibiting itih1 mRNA expression

After treating the HepG2 cells with the *Gentiana triflora Pallas* extract, real-time polymerase chain reaction (quantitative PCR, qPCR) was performed as follows in order to confirm an effect of inhibiting *itih1* mRNA expression.

The cells were cultured in a cell culture medium containing high concentration (25 mM) glucose for 24 hours, and then the *Gentiana triflora Pallas* whole plant, root extract was diluted in the culture solution to a final extraction concentration of 10 µg/ml, supplied to the cell line, and cultured for 24 hours. After 24 hours, the medium was removed and washed with PBS, and then the attached cells were scraped off with a scraper and collected in a centrifuge. Total RNA was extracted from the cells using a miRNA extraction kit (mirVana miRNA Isolation Kit), and cDNA was synthesized using a ReverTra Ace qPCR RT Master Mix with gDNA Remover, and then it was used as a template DNA to confirm the transcript expression level. In order to confirm the expression of *itih1* mRNA, primer pairs of itih1-qpcr1 and itih1-qpcr2 were used, and qPCR reactions were performed using primer pairs of gapdh-qpcr1 and gapdh-qpcr2 as a reference gene, respectively. It was confirmed whether or not the gene sequence was amplified at the transcript level. The sequences of each primer pair are shown in Table 1 below.

**[Table 1]**

| Primer | Sequence (5' → 3' direction) |
|---|---|
| itih1-qpcr1 (SEQ ID NO: 1) | TGGACACTTCAGTCAATGGTGT |
| itih1-qpcr2 (SEQ ID NO: 2) | GATGAAGGCTGTCTTGGGGA |
| gapdh-qpcr1 (SEQ ID NO: 3) | AGGGCTGCTTTTAACTCTGGT |
| gapdh-qpcr2 (SEQ ID NO: 4) | CCCCACTTGATTTTGGAGGGA |

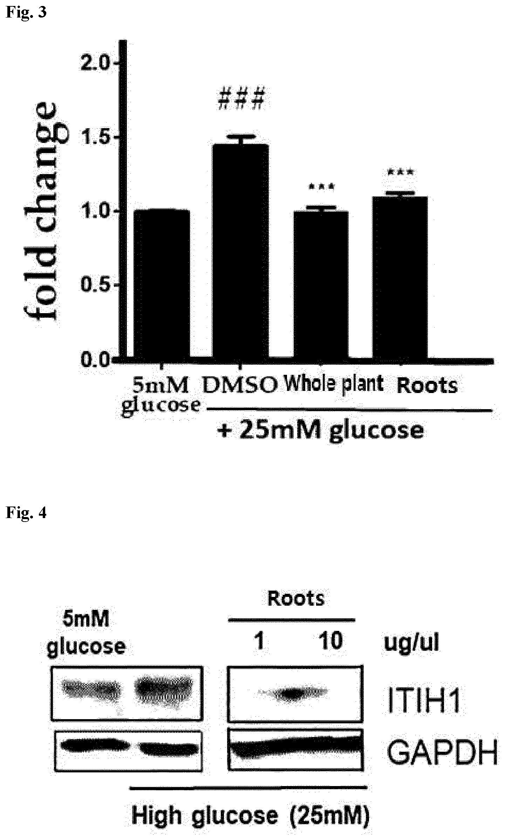

Figure 3 shows the expression level of *itih1* mRNA in the cells treated with the *Gentiana triflora Pallas* whole plant and root extracts.

As confirmed in Figure 3, it was confirmed that *itih1* mRNA expression in the case of treatment with the *Gentiana triflora Pallas* extract was significantly reduced compared to the high concentration glucose condition without treatment with the extract, and it was recovered to a level similar to that of the normal group not treated with high concentration glucose.

### [Example 4]

### Confirmation of effect of Gentiana triflora Pallas extract on inhibiting ITIH1 protein expression

In the cells obtained in Example 3, the expression of ITIH1 protein was confirmed by Western blotting. Specifically, the cell lysates were obtained by lysing the cells with a cell lysis buffer (RIPA lysis buffer (25 mM Tris-HCl pH 7.6, 150 mM NaCl, 1% NP-40, 1% sodium deoxycholate, 0.1% SDS), 120 mM, Protease Inhibitor Cocktail (AEBSF, Aprotinin, Bestatin, E64, Leupeptin, and Pepstatin A), Phosphatase Inhibitor Cocktail (sodium fluoride, sodium pyrophosphate, β-glycerophosphate, and sodium orthovanadate)), and the cell lysates were mixed with a sample buffer (60 mM Tris-HCl (pH 6.8), 25% glycerol, 0.1% bromophenol blue, 2% SDS, 2% mercapto ethanol) and cultured for 10 minutes on a heating block at 100 °C. The supernatant was collected by centrifugation at 13,000 rpm, 4 °C for 5 minutes, and each sample was loaded into an SDS PAGE gel (12%) and electrophoresed at 120 V. The entire protein separated in the SDS PAGE gel was transferred to the NC membrane, and then ITIH1 protein expression was confirmed using an antibody against ITIH1 (matured form) and β-actin, and the results are shown in Figure 4, and the results of quantifying the protein band in Figure 4 are shown in Table 2 below.

**[Table 2]**

| Item | Fold value |
|---|---|
| Untreated group | 1.03 |
| High concentration glucose treated group | 1.54 |
| High concentration glucose + *Gentiana triflora Pallas* root 1 µg/ml | 0.63 |
| High concentration glucose + *Gentiana triflora Pallas* root 10 µg/ml | 0.39 |

As shown in Figure 4 and Table 2, when cell lines in which ITIH1 protein expression was increased by more than 1.5 times in a medium containing high concentration glucose were treated with the *Gentiana triflora Pallas* root extract at concentrations of 1 and 10 µg/ml, respectively, it was confirmed that the amount of ITIH1 protein was significantly reduced.

As a result, it can be seen that the *Gentiana triflora Pallas* extract has an effect of improving insulin sensitivity and an effect of treating diseases related to insulin resistance by excellently inhibiting both mRNA expression and protein expression of ITIH1, which lowers insulin sensitivity in a hyperglycemic environment.

### [Example 5]

### Confirmation of increased expression of ogt mRNA under high concentration glucose conditions

It is known that the increase in OGT (O-GlcNAc transferase) due to the decrease in G13 (G protein alpha-13) in hyperglycemic conditions increases the stability of ITIH1, increasing the intracellular concentration and secretion amount of ITIH1. The evaluation method for *ogt* mRNA inhibitory efficacy was confirmed as follows.

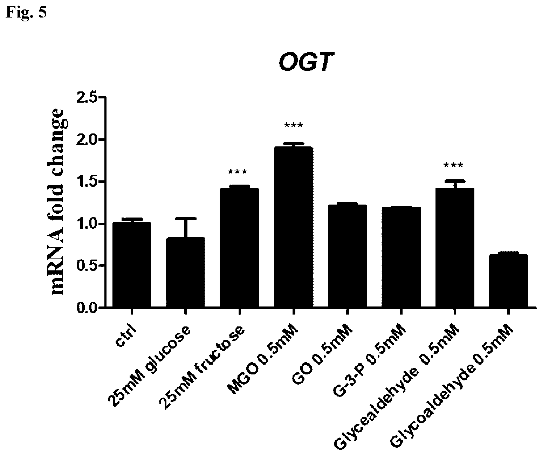

The HepG2 cells were cultured in a cell culture medium containing glucose, fructose, methylglyoxal (MGO), glyoxal (GO), glyceraldehyde 3-phosphate (G3P), glyceraldehyde, and glycolaldehyde for 24 hours, respectively, and the expression level of *ogt* mRNA was measured and shown in Figure 5.

As shown in Figure 5, when treated with MGO known to cause diabetes and diabetic complications as a precursor of advanced glycation end products, the *ogt* mRNA expression was increased nearly twice as compared to the control group. Therefore, in the following, the expression of *ogt* mRNA according to the treatment with the *Gentiana triflora Pallas* extract was confirmed in the HepG2 cells under high concentration glucose and MGO treatment conditions.

### [Example 6]

### Confirmation of effect of Gentiana triflora Pallas extract on inhibiting ogt mRNA expression

After treating HepG2 cells with the *Gentiana triflora Pallas* extract, real-time polymerase chain reaction (quantitative PCR, qPCR) was performed as follows in order to confirm an effect of inhibiting *ogt* mRNA expression.

The HepG2 cells were cultured in a cell culture medium containing high concentration (25 mM) glucose for 24 hours, and then 500 µM MGO was induced for 1 hour. After treatment, the *Gentiana triflora Pallas* root extract was diluted in the culture solution to a final extraction concentration of 10 µg/ml, supplied to the cell line, and cultured for 24 hours. After 24 hours, the medium was removed and washed with PBS, and then the attached cells were scraped off with a scraper and collected in a centrifuge. Total RNA was extracted from the cells using a miRNA extraction kit (mirVana miRNA Isolation Kit), and cDNA was synthesized using a ReverTra Ace qPCR RT Master Mix with gDNA Remover, and then it was used as a template DNA to confirm the transcript expression level. In order to confirm the expression of *ogt* mRNA, primer pairs of ogt-qpcr1 and ogt-qpcr2 were used, and qPCR reactions were performed using primer pairs of gapdh-qpcr1 and gapdh-qpcr2 as a reference gene, respectively. It was confirmed whether or not the gene sequence was amplified at the transcript level. The sequences of each primer pair are shown in Table 3 below.

**[Table 3]**

| Primer | Sequence (5' → 3' direction) |
|---|---|
| ogt-qpcr1 (SEQ ID NO: 5) | CTTTAGCACTCTGGCAATTAAACAG |
| ogt-qpcr2 (SEQ ID NO: 6) | TCAAATAACATGCCTTGGCTTC |
| gapdh-qpcr1 (SEQ ID NO: 3) | AGGGCTGCTTTTAACTCTGGT |
| gapdh-qpcr2 (SEQ ID NO: 4) | CCCCACTTGATTTTGGAGGGA |

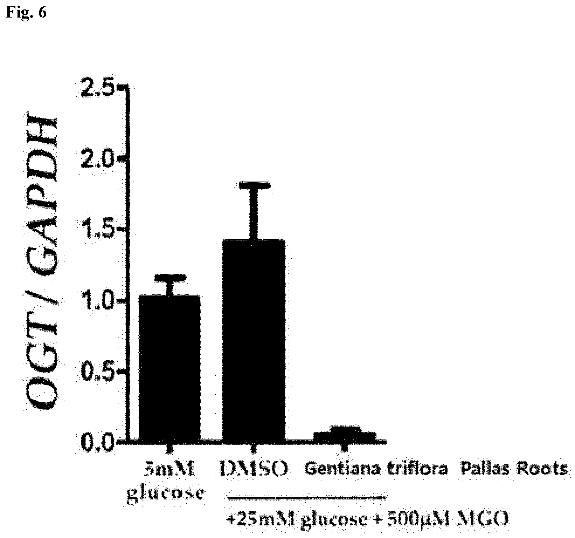

Figure 6 shows the expression level of *ogt* mRNA in the cells treated with the *Gentiana triflora Pallas* root extract.

As shown in Figure 6, it was confirmed that the expression of *ogt* mRNA in the high concentration glucose and MGO treated group was increased by 1.41 times compared to the normal group, but the expression of *ogt* mRNA in the group treated with the *Gentiana triflora Pallas* root extract was reduced to 0.03 times that of the normal group, indicating a significant OGT inhibitory effect.

As a result, it can be seen that the *Gentiana triflora Pallas* extract has an effect of improving insulin sensitivity and an effect of treating diseases related to insulin resistance by significantly reducing OGT, which acts to increase ITIH1.

## Claims

1. A pharmaceutical composition for improving insulin sensitivity, comprising a *Gentiana triflora Pallas* extract.

2. The pharmaceutical composition for improving insulin sensitivity according to claim 1, wherein the pharmaceutical composition has an effect of increasing insulin sensitivity and reducing insulin resistance.

3. The pharmaceutical composition according to claim 1, wherein the pharmaceutical composition is for the prevention or treatment of at least one disease selected from the group consisting of diabetes, diabetic complications, impaired glucose tolerance, and insulin resistance syndrome.

4. The pharmaceutical composition according to claim 3, wherein the diabetic complications are at least one disease selected from the group consisting of diabetic retinopathy, diabetic cataract, diabetic nephropathy, and diabetic neuropathy.

5. The pharmaceutical composition according to claim 3, wherein the insulin resistance syndrome is at least one disease selected from the group consisting of obesity, hypertension, arteriosclerosis, hyperlipidemia, hyperinsulinemia, non-alcoholic fatty liver, and type 2 diabetes caused by insulin resistance.

6. The pharmaceutical composition according to claim 1, wherein the *Gentiana triflora Pallas* extract is a *Gentiana triflora Pallas* whole plant extract or a *Gentiana triflora Pallas* root extract.

7. The pharmaceutical composition according to claim 1, wherein the *Gentiana triflora Pallas* extract reduces the expression of ITIH1 (Inter-alpha Trypsin Inhibitor Heavy chain 1) mRNA or protein.

8. The pharmaceutical composition according to claim 1, wherein the *Gentiana triflora Pallas* extract reduces the expression of *ogt* (O-GlcNAc Transferase) mRNA.

9. A food composition for improving insulin sensitivity, comprising a *Gentiana triflora Pallas* extract.

10. The food composition for improving insulin sensitivity according to claim 9, wherein the food composition has an effect of increasing insulin sensitivity and reducing insulin resistance.

11. The food composition according to claim 9, wherein the food composition is for the prevention or improvement of at least one disease selected from the group consisting of diabetes, diabetic complications, impaired glucose tolerance, and insulin resistance syndrome.

12. An animal feed composition for improving insulin sensitivity, comprising a *Gentiana triflora Pallas* extract.

13. The animal feed composition for improving insulin sensitivity according to claim 12, wherein the animal feed composition has an effect of increasing insulin sensitivity and reducing insulin resistance.

14. The animal feed composition according to claim 12, wherein the animal feed composition is for the prevention or improvement of at least one disease selected from the group consisting of diabetes, diabetic complications, impaired glucose tolerance, and insulin resistance syndrome.
